## Europäisches Patentamt
## European Patent Office
## Office européen des brevets

(19)

(11) Publication number: **0 057 041**
**B1**

(12) **EUROPEAN PATENT SPECIFICATION**

(45) Date of publication of patent specification: **09.10.85**

(21) Application number: **82200076.6**

(22) Date of filing: **22.01.82**

(51) Int. Cl.⁴: **B 01 J 27/30, C 07 C 5/48,**
**C 07 C 120/00, C 07 B 35/04,**
**C 07 B 43/08, C 07 B 41/00**

(54) Catalyst reactivation.

(30) Priority: **27.01.81 NL 8100364**

(43) Date of publication of application:
**04.08.82 Bulletin 82/31**

(45) Publication of the grant of the patent:
**09.10.85 Bulletin 85/41**

(84) Designated Contracting States:
**AT BE DE FR GB IT NL**

(56) References cited:
**DE-A-2 948 771**
**FR-A-2 280 428**
**GB-A-2 010 113**

**J.C. BAILAR "Comprehensive inorganic chemistry", 1st ed., vol. 2, Pergamon Press, Oxford, GB, pages 971-974,986**

(73) Proprietor: **STAMICARBON B.V.**
**Postbus 10**
**NL-6160 MC Geleen (NL)**

(72) Inventor: **Kleuskens, Engelina Catharina**
**Gruijterstraat 1**
**NL-6171 TL Stein (NL)**
Inventor: **van de Mond, Theodorus Johannes**
**Oranjelaan 10**
**NL-6166 BR Geleen (NL)**
Inventor: **Maessen, Johannes Gerardus**
**Hendrikus**
**Karthuizerlaan 17**
**NL-6042 NB Roermond (NL)**

(74) Representative: **Leherte, Georges Maurice**
**Lucien Marie et al**
**Octrooibureau DSM P.O.Box 9**
**NL-6160 MA Geleen (NL)**

Courier Press, Leamington Spa, England.

# 0 057 041

## Description

The invention relates to a process for the reactivation of oxidic catalysts containing tellurium.

As is known, olefines can be oxidized, ammoxidized or oxidatively dehydrogenated with the aid of an oxidic catalyst, the catalytically active components of which consist mainly of oxides of one or more of the metals iron and/or antimony, preferably together with oxides of one or more of the metals molybdenum, vanadium and tungsten, and one or more oxides of metals such as zinc, tin, cobalt, nickel, magnesium, copper, lanthanum, bismuth, phosphorus and borium, which catalyst also contains a tellurium oxide as promotor. Such catalysts are described, *inter alia*, NL—A 7013058, 7501472, 7703868 and DE—A 2534467.

The productivity of the catalyst decreases by use. Productivity is here understood to mean the product of the conversion and the selectivity towards the desired product. This deterioration, which is normal for catalysts, can even be accelerated if the reaction conditions are not optimum.

Various processes have been proposed for the reactivation of such iron and/or antimony containing oxide catalysts, for instance in NL—A 78.10685, 78.11147 and 79.08750. A disadvantage of these known methods is that the catalyst to be reactivated is to be removed from the reactor and subjected to a series of operations.

The object of the invention is to find a process for the reactivation of the spent catalyst which preferably is carried out in the simplest way possible.

According to the invention a spent oxidic catalyst that is suitable for oxidation, ammoxidation and/or oxidative dehydrogenation of olefines and that contains a tellurium oxide and as active components mainly oxides of antimony and/or iron is reactivated by mixing the spent catalyst with a solid particulate tellurium compound or with a particulate, solid carrier material containing a tellurium compound, in an amount that is sufficient to raise the tellurium content (calculated as metal) of the reactivated catalyst to a value between 90% and 500% of the tellurium content of the original catalyst.

The applicant has found that a decrease in the productivity of the catalyst is attended by a reduction of the tellurium content. This does not need to imply that there is a direct causal connection between these two phenomena. The applicant did find that the productivity, and particularly the selectivity, is increased by adding a tellurium compound.

The catalyst generally is reactivated when the productivity has decreased by between 5% and 10% of the orginal value. The catalyst generally will be reactivated when the tellurium content still is at least 50%, and preferably at least 75%, of the original tellurium content. It is, however, also possible to further exhaust the catalyst before reactivating it, or conversely, to reactivate it sooner, if this is more advantageous from an economic point of view.

By preference tellurium oxide is used as tellurium compound, or a compound which is easily converted into a tellurium oxide under the conditions of use or in the process of calcination at a temperature between 600°C and 900°C.

In particular the use of tellurium oxide is preferred. The advantage of this method is that the added solid tellurium compound can easily be mixed with the catalyst to be reactivated. In the case of catalysts that are used in fluidized condition it is possible to simply add the solid tellurium compound to the catalyst bed, without removing the catalyst to be reactivated from the reactor. The application of tellurium oxide on a carrier material has an additional advantage that the particles can more easily be processed in this shape and that particles can be prepared that have fluidization properties that agree with the fluidization properties of the catalyst to be regenerated. The tellurium content of such carrier material containing tellurium oxide may be between 5% and 80%. Optionally other metal oxides may be present besides tellurium oxide. That the addition of tellurium oxide, on a carrier or not, produces an effect is remarkable, because under the reaction conditions tellurium oxide is hardly volatile and therefore migration of the tellurium in one form or another is not to be expected.

In general such an amount of tellurium compound is added that the tellurium content (calculated as metal) of the reactivated catalyst is between 90% and 500% of the original tellurium content. By preference such an amount is added that the tellurium content is between 90% and 200%, and more in particular between 95% and 150%, of the original tellurium content. Reactivated catalyst is here understood to include the mixture of spent catalyst and tellurium oxide, on a carrier or not.

The process generally can be applied to tellurium containing oxidic catalysts that are used for oxidation or ammoxidation or oxidative dehydrogenation of olefines.

Examples of catalysts that are suitable especially for the oxidation and ammoxidation of olefines are the oxidic catalysts on an iron-antimony oxide basis that have as essential components iron, antimony and tellurium, one or more of the elements vanadium, molybdenum and tungsten, and optionally one or more of the elements copper, magnesium, zinc, lanthanum, cerium, aluminium, chromium, manganese, cobalt, nickel, bismuth, tin phosphorus and borium. The overall composition of these catalysts, apart from the carrier material, is $Fe_{10}Sb_aTe_bMe_cX_dO_e$, where Me is at least one of the elements vanadium, molybdenum and tungsten, X is at least one of the elements copper, magnesium, zinc, lanthanum, cerium, aluminium, chromium, manganese, cobalt, nickel, bismuth, tin, phosphorus and borium, a is 5—80, b is 0.1—10, c is 0.01—10 and d is 0—20, where e is the number of oxygen atoms corresponding with an oxydic structure.

The invention will be elucidated on the basis of an example.

2

**0 057 041**

Example I

Propylene was converted into acrylonitrile by passing a gaseous mixture of propylene, ammonia and air through a fluidized catalyst bed at a pressure of 1 atm. and a temperature of 430°C. The gaseous mixture consisted of 7.7 vol.% of propylene, 8.36 vol.% of ammonia and 17.54 vol.% of oxygen, the remainder being nitrogen. The gaseous mixture was passed through at a linear rate of 1.79 cm/sec. The total amount of gas passed through per hour was 15.7 l. In test no. 1 the reactor contained 40 g of a commercially available catalyst (NS 733-b catalyst from Nitto Chemical Company), the productivity of which had decreased by use in a commercial acrylonitrile production unit. The tellurium content (calculated as metal) of this catalyst was about 83% of that of the fresh catalyst. In tests Nos. 2 through 6 a catalyst was used that had been obtained by mixing 40 g of the above-mentioned catalyst with 0.2 g tellurium dioxide. Using a gas chromatograph, the composition of the reaction gas was determined at the reactor outlet, so that the propylene conversion and the selectivity towards acrylonitrile could be calculated. The results are tabulated below.

| Test No. | 1 | 2 | 3 | 4 | 5 | 6 |
|---|---|---|---|---|---|---|
| time after start, hours | — | $\frac{1}{2}$ | 2 | 3 | 4 | 24 |
| propylene conversion, % | 95.4 | 95.0 | 95.5 | 95.5 | 95.7 | 95.9 |
| selectivity towards acrylonitrile, % | 74.3 | 77.1 | 76.8 | 77.2 | 77.2 | 77.6 |
| productivity | 70.9 | 73.3 | 73.4 | 73.8 | 73.9 | 74.5 |

**Claims**

1. Process for the reactivation of a spent oxidic catalyst that is suitable for oxidation, ammoxidation and/or oxidative dehydrogenation of olefines and that contains a tellurium oxide and as active components mainly oxides of antimony and/or iron, this process being characterized in that the spent catalyst is mixed with a solid particulate tellurium compound or with a particulate, solid carrier material containing a tellurium compound, in an amount that is sufficient to raise the tellurium content (calculated as metal) of the reactivated catalyst to a value between 90% and 500% of the tellurium content of the original, fresh catalyst.

2. Process according to claim 1, characterized in that such an amount of tellurium compound is added that the tellurium content reaches a value of 90% to 200% of the tellurium content of the original catalyst.

3. Process according to claims 1—2, characterized in that such an amount of tellurium compound is added that the tellurium content reaches a value of 95% to 150% of the tellurium content of the original catalyst.

4. Process according to claims 1—2, characterized in that as tellurium compound use is made of tellurium oxide or a compound that is easily converted into a tellurium oxide under the conditions of use or by calcination at a temperature between 600°C and 900°C.

5. Process according to claims 1—4, characterized in that the catalyst is reactivated by mixing the spent catalyst with tellurium oxide.

6. Process according to claims 1—4, characterized in that the catalyst is reactivated by mixing the spent catalyst with tellurium oxide that has been applied on a solid particulate carrier material.

7. Process according to claims 1—6, characterized in that an oxide catalyst is reactivated which, apart from the carrier material, satisfies the formula $Fe_{10}Sb_aTe_bMe_cX_dO_e$, where Me is at least one of the elements vanadium, molybdenum and tungsten, X is at least one of the elements copper, magnesium, zinc, lanthanum, cerium, aluminium, chromium, manganese, cobalt, nickel bismuth, tin, phosphorus and borium, a is 5—80, b is 0.1—10, c is 0.01—10 and d is 0—20, while e is the number of oxygen atoms corresponding with an oxidic structure.

8. Process for the oxidation, ammoxidation or oxidative dehydrogenation of an olefine using an oxidic catalyst containing tellurium which has as active components mainly oxides of antimony and/or iron, characterized in that a catalyst is used which has been reactivated by the process according to one or more of the claims 1—7.

9. Process for the oxidation or ammoxidation of an olefine using a catalyst of the formula $Fe_{10}Sb_aTe_bMe_cX_dO_e$, where Me is at least one of the elements vanadium, molybdenum and tungsten, X is one of the elements copper, magnesium, zinc, lanthanum, cerium, aluminium, chromium, manganese, cobalt, nickel, bismuth, tin, phosphorus and borium, a is 5—80, b is 0.1—10, c is 0.01—10, d is 0 to 20 and e is the number of oxygen atoms satisfying an oxidic structure, this process being characterized in that a catalyst is used which consists of a mixture of particles of a spent catalyst of the composition given above and particles of tellurium oxide or particles of a solid carrier material on which tellurium oxide is present.

3

**Revendications**

1. Procédé de réactivation d'un catalyseur usé d'oxydation, convenant pour l'oxydation, l'ammoxydation et/ou la déshydrogénation oxydante des oléfines, et qui contient un oxyde de tellure et, comme composants actifs, principalement des oxydes d'antimoine et/ou de fer, ce procédé étant caractérisé en ce qu'on mélange le catalyseur usé avec des particules solides d'un composé de tellure, ou avec un support solide particulaire contenant un composé de tellure, en une quantité suffisante pour élever la teneur en tellure (calculée en métal) du catalyseur réactivé, à une valeur comprise entre 90% et 500% de la teneur en tellure du catalyseur frais, originel.

2. Procédé selon la revendication 1, caractérisé en ce qu'on ajoute le composé de tellure en quantité telle que la teneur en tellure atteint une valeur de 90% à 200% de la teneur en tellure du catalyseur originel.

3. Procédé selon l'une des revendications 1 et 2, caractérisé en ce qu'on ajoute le composé de tellure en quantité telle que la teneur en tellure atteint une valeur de 95% à 150% de la teneur en tellure du catalyseur originel.

4. Procédé selon l'une des revendications 1 et 2, caractérisé en ce qu'on utilise comme composé de tellure un oxyde de tellure, ou un composé aisément convertible en oxyde de tellure, dans les conditions d'emploi, ou par calcination à une température comprise entre 600°C et 900°C.

5. Procédé selon l'une des revendications 1 à 4, caractérisé en ce qu'on réactive le catalyseur par mélange du catalyseur usé avec l'oxyde de tellure.

6. Procédé selon l'une des revendications 1 à 4, caractérisé en ce qu'on réactive le catalyseur par mélange du catalyseur usé avec de l'oxyde de tellure qui est appliqué sur un support solide particulaire.

7. Procédé selon l'une des revendications 1 à 6, caractérisé en ce qu'on réactive un catalyseur d'oxydation qui, à part le support, répond à la formule $Fe_{10}Sb_aTe_bMe_cX_dO_e$, où Me est au moins l'un des éléments vanadium, molybdène et tungstène, X est au moins l'un des éléments cuivre, magnésium, zinc, lanthane, cérium, aluminium, chrome, manganèse, cobalt, nickel, bismuth, étain, phosphore et bore, $a = 5—80$, $b = 0,1—10$, $c = 0,01—10$ et $d = 0—20$, tandis que e est le nombre d'atomes d'oxygène correspondant à une structure oxydante.

8. Procéde pour l'oxydation, l'ou la déshydrogénation oxydante des oléfines, utilisant un catalyseur d'oxydation contenant du tellure, comportant comme composants actifs principalement des oxydes d'antimoine et/ou fer, caractérisé en ce qu'on utilise un catalyseur qui a été réactivé suivant le procédé selon l'une des revendications 1 à 7.

9. Procédé d'oxydation ou d'ammoxydation des oléfines, utilisant un catalyseur de formule $Fe_{10}Sb_aTe_bMe_cX_dO_e$, dans laquelle Me est au moins l'un des éléments vanadium, molybdène et tungstène, X est au moins l'un des éléments cuivre, magnésium, zinc, lanthane, cérium, aluminium, chrome, manganèse, cobalt, nickel, bismuth, étain, phosphore et bore, $a = 5—80$, $b = 0,1—10$, $c = 0,01—10$, $d = 0—20$, et e est le nombre d'atomes d'oxygène répondant à une structure oxydante, ce procédé étant caractérisé en ce qu'on utilise un catalyseur qui se compose d'un mélange de particules d'un catalyseur usé de la composition donnée ci-dessus, et de particules d'oxyde de tellure, ou de particules d'un support solide, sur laquelle l'oxyde de tellure est présent.

**Patentansprüche**

1. Verfahren zur Reaktivierung eines verbrauchten oxidischen Katalysators, der für die Oxidation, Ammoxidation und/oder oxidative Dehydrierung von Olefinen geeignet ist und der ein Telluroxid und als aktive Komponenten hauptsächlich Oxide von Antimon und/oder Eisen enthält, dadurch gekennzeichnet, daß der verbrauchte Katalysator mit einer festen, teilchenförmigen Tellurverbindung oder mit einem eine Tellurverbindung enthaltenden, teilchenförmigen festen Trägermaterial in einer Menge vermischt wird, die ausreicht, um den Tellurgehalt (berechnet als Metall) des reaktivierten Katalysators auf einen Wert zwischen 90 und 500% des Tellurgehalts des ursprünglichen, frischen Katalysators zu erhöhen.

2. Verfahren nach Anspruch 1, dadurch gekennzeichnet, daß eine solche Menge Tellurverbindung zugesetzt wird, daß der Tellurgehalt 90 bis 200% des Tellurgehalts des ursprünglichen Katalysators erreicht.

3. Verfahren nach den Ansprüchen 1—2, dadurch gekennzeichnet, daß eine solche Menge Tellurverbindung zugesetzt wird, daß der Tellurgehalt 95 bis 150% des Tellurgehalts des ursprünglichen Katalysators erreicht.

4. Verfahren nach den Ansprüchen 1—2, dadurch gekennzeichnet, daß als Tellurverbindung Telluroxid oder eine Verbindung verwendet wird, die unter den Gebrauchsbedingung oder durch Calcinierung bei einer Temperatur zwischen 600 und 900°C leicht in ein Telluroxid übergeführt wird.

5. Verfahren nach den Ansprüchen 1—4, dadurch gekennzeichnet, daß der Katalysator durch Vermischen des verbrauchten Katalysators mit Telluroxid reaktiviert wird.

6. Verfahren nach den Ansprüchen 1—4, dadurch gekennzeichnet, daß der Katalysator durch Vermischen des verbrauchten Katalysators mit Telluroxid, das auf ein festes teilchenförmiges Trägermaterial aufgebracht worden ist, reaktiviert wird.

7. Verfahren nach den Ansprüchen 1—6, dadurch gekennzeichnet, daß ein oxidischer Katalysator reaktiviert wird, der, abgesehen vom Trägermaterial, der Formel $Fe_{10}Sb_aTe_bMe_cX_dO_e$ entspricht, worin Me

mindestens eines der Elemente Vanadium, Molybdän und Wolfram, X mindestens eines der Elemente Kupfer, Magnesium, Zink, Lanthan, Cer, Aluminium, Chrom, Mangan, Kobalt, Nickel, Wismuth, Zinn, Phosphor und Bor, a 5—80, b 0,1—10, c 0,01—10 und d 0—20 ist, während e die Zahl der Säurestoffatome bedeutet, die einer oxidischen Struktur entspricht.

8. Verfahren zur Oxidation, Ammoxidation oder oxidativen Dehydrierung eines Olefins unter Verwendung eines Tellur enthaltenden oxidischen Katalysators, welcher als aktive Komponenten hauptsächlich Oxide von Antimon und/oder Eisen enthält, dadurch gekennzeichnet, daß ein Katalysator verwendet wird, der nach dem Verfahren gemäß einem oder mehreren der Ansprüche 1—7 reaktiviert worden ist.

9. Verfahren zur Oxidation oder Ammoxidation eines Olefins unter Verwendung eines Katalysators der Formel $Fe_{10}Sb_aTe_bMe_cX_dO_e$, worin Me mindestens eines der Elemente Vanadium, Molybdän und Wolfram, X mindestens eines der Elemente Kupfer, Magnesium, Zink, Lanthan, Cer, Aluminium, Chrom, Mangan, Kobalt, Nickel, Wismuth, Zinn, Phosphor und Bor, a 5—80, b 0,1—10, c 0,01—10 und d 0—20 ist, während e die Zahl der Säurestoffatome bedeutet, die einer oxidischen Struktur entspricht, dadurch gekennzeichnet, daß ein Katalysator verwendet wird, welcher aus einem Gemisch von Teilchen eines verbrauchten Katalysators mit der obigen Zusammensetzung und Teilchen von Telluroxid oder Teilchen eines festen Trägermaterials, auf welchen Telluroxid vorhanden ist, besteht.